# FASCICULE DE BREVET EUROPEEN

(11) **EP 2 794 638 B1**
(45) Date de publication et mention de la délivrance du brevet: **27.05.2020**
(21) Numéro de dépôt: 12816737.6
(22) Date de dépôt: 19.12.2012
(51) Int. Cl.: C07K 14/18, G01N 33/569

(54) **PROCEDE DE DETECTION D'UNE INFECTION PAR LE VIRUS DE L'HEPATITE C**
VERFAHREN ZUM NACHWEIS EINER INFEKTION DURCH HEPATITIS C VIRUS
METHOD FOR DETECTING AN INFECTION BY THE HEPATITIS C VIRUS

(30) Priorité: 20.12.2011 FR 1161981
(43) Date de publication de la demande: 29.10.2014
(73) Titulaire: Bio-Rad Europe GmbH, 4051 Basel (CH)
(72) Inventeur: GADELLE, Stéphane, F-91430 Vauhallan (FR); RIEUNIER, François, F-78390 Bois D'arcy (FR)
(74) Mandataire: Chajmowicz, Marion
(86) Numéro de dépôt international: PCT/FR2012/053001
(87) Numéro de publication internationale: WO 2013/093345

(56) Documents cités:
- WO-A1-00/31130
- WO-A2-03/095968
- WO-A2-2008/070727
- F. ANSALDI ET AL: "Combination hepatitis C virus antigen and antibody immunoassay as a new tool for early diagnosis of infection", JOURNAL OF VIRAL HEPATITIS, vol. 13, no. 1, 1 janvier 2006 (2006-01-01), pages 5-10, XP055030213, ISSN: 1352-0504, DOI: 10.1111/j.1365-2893.2005.00646.x
- ALHUSAIN J. ALZAHRANI: "Simultaneous detection of hepatitis C virus core antigen and antibodies in Saudi drug users using a novel assay", JOURNAL OF MEDICAL VIROLOGY, vol. 80, no. 4, 1 avril 2008 (2008-04-01), pages 603-606, XP055029920, ISSN: 0146-6615, DOI: 10.1002/jmv.21075 -& Laura Dean and Keith Perry: "Evaluation of Murex HCV Ag/Ab combination", Heath protection Agency , 1 février 2007 (2007-02-01), XP002678171, Extrait de l'Internet: URL:http://www.hpa.org.uk/webc/HPAwebFile/ HPAweb_C/1317133424210 [extrait le 2012-06-19]

## Description

L'invention concerne la détection in vitro d'une infection par un virus de l'hépatite C (VHC), et l'utilisation, à cette fin, d'un peptide antigénique dérivé de la protéine de capside du virus.

### Arrière-plan technologique de l'invention :

L'infection par le virus de l'hépatite C est un problème de santé préoccupant et reconnu depuis longtemps, en particulier en transfusion sanguine.

Pour réduire les risques post-transfusionnels, il est nécessaire de détecter la présence du virus lui-même, avant l'apparition d'anticorps, et le plus tôt possible après la contamination. Cette période entre la contamination et la séroconversion (c'est à dire l'apparition d'anticorps) est appelée « fenêtre sérologique ».

Dans l'optique de disposer d'une méthode simple, sensible, spécifique, reproductible, bon marché et de mise en oeuvre facile, automatisable, en vue du dépistage de masse-pour détecter, en premier lieu, l'antigène VHC pendant la période de la fenêtre sérologique, puis suivre l'évolution sérologique du patient après la séroconversion, une détection des anticorps anti-VHC, associée à une détection d'un antigène de VHC, a été proposée.

Cependant, ceci pose un problème majeur, celui de l'interférence entre les anticorps anti-VHC présents dans le sérum et des anticorps anti-VHC marqués. Ainsi l'introduction sur une phase solide, en vue de la détection d'un anticorps donné, d'un antigène cible qui aurait les mêmes épitopes que ceux reconnus par le(s) anticorps marqué(s), utilisé(s) en vue de la détection simultanée en sandwich d'un antigène, entraînerait irrémédiablement une fixation d'anticorps marqué s) sur la phase solide et donc une réponse faussement positive de l'essai.

Ceci est particulièrement vrai dans un système de détection simultanée, sur la même phase solide, d'anticorps anti-capside du VHC et d'antigène de capside du VHC. Ainsi le dépôt sur la phase solide, en vue de la détection d'anticorps anti-capside du VHC, d'un antigène de capside qui a les mêmes épitopes que ceux reconnus par le(s) anticorps anti-capside VHC marqué(s) utilisé(s) en vue de la détection de l'antigène capsidique entraîne une fixation d'anticorps marqué(s) sur la phase solide et résulte en une réponse faussement positive de l'essai.

Face au problème des interférences, la demande EP 1 020 727 (Advanced Life Science Institute) propose une méthode de mesure simultanée de l'antigène de capside VHC et des anticorps anti-capside VHC (un essai de type « Combo »), dans laquelle l'antigène est capturé et marqué par des anticorps dirigés contre des épitopes capsidiques différents des épitopes capsidiques servant simultanément à la capture et la révélation ou détection d'anticorps anti-capside. Un exemple représentatif est donné où, dans l'essai simultané de détection de l'antigène en sandwich et des anticorps en essai indirect, on utilise, pour détecter l'antigène, un premier anticorps (de capture), dirigé contre les épitopes de la séquence de l'acide aminé (AA) 100 à l'acide aminé 130 de la capside du VHC, et un deuxième anticorps (de détection), dirigé contre les épitopes de la séquence AA40-50, et, pour détecter les anticorps, l'antigène de capture utilisé contient pour sa part les séquences AA1-42 et AA66-80.

La demande de brevet WO 01/96875 (CHIRON) décrit, entre autres, un essai de détection simultanée de la capside et des anticorps anti NS3 et NS4 faisant usage de N- laurylsarcosine comme détergent. Elle évoque un essai de détection simultanée de l'antigène de capside (en sandwich) et des anticorps anti- capside et anti-protéines non-structurales du VHC (en sandwich double antigène). On utilise, pour capturer l'antigène, deux anticorps, c11-3 et c11-7, réputés reconnaître une vaste portion N-terminale (AA 10-53) de la capside du VHC, et pour la détection un troisième anticorps, c11-14, réputé reconnaître une portion C-terminale (AA 120-130) de la capside du VHC. Pour détecter les anticorps, l'antigène de capture utilisé est un antigène de fusion à épitopes multiples qui contient, en fusion avec un fragment de superoxyde-dismutase (« SOD »), des antigènes NS3, NS4, NS5 et des séries de séquences capsidiques de plusieurs souches de VHC : AA 9-53, porteuse de la mutation R47L, AA 64-88 et AA 67-84.

La demande de brevet EP 1 251 353 (Ortho-Clinical Diagnostics) décrit un essai «Combo complet » utilisant les mêmes anticorps pour la détection de la capside, sans toutefois préciser leur origine ni leur spécificité épitopique. La détection des anticorps anti-capside se fait à l'aide d'un antigène de capside modifié (par mutagenèse) : C22KSNV47, 48 (protéine de fusion avec la SOD comprenant la séquence capsidique AA 10-99 délétée des acides aminés 47 et 48) ou C22KSR47L (protéine de fusion avec la SOD comprenant la séquence capsidique AA 10-99, avec une leucine remplaçant une arginine en position 47).

La demande de brevet WO2003/002749 (Abbott) décrit de nombreux antigènes et essais de détection d'antigène capsidique du VHC. Le seul essai « Combo complet » qu'elle décrit-sous le nom de « Real Combo » fait usage, pour la détection des anticorps anti capside, d'un peptide biotinylé correspondant aux acides aminés 11-28 de la capside, immobilisé en phase solide. Pour la détection de la capside, elle met en oeuvre la combinaison d'anticorps d'Advanced Life Science Institute C11-14 (reconnaissant la séquence capsidique AA 45-50) en phase solide et C11-10 (reconnaissant la séquence capsidique AA 32-36) marqué à l'acridine. La demande WO 03/002 749 réalise donc la capture de l'antigène capsidique et la capture des anticorps anti-capside par le biais de deux sites capsidiques clairement distincts, i. e. non superposés, (AA 11-28 pour détecter les anticorps et anticorps anti AA 32-36 et AA 45-50 pour détecter l'antigène).

La demande de brevet EP 1 310 512 (Ortho-Clinical Diagnostics) décrit l'utilisation de peptides contenant une séquence de capside mutée, éliminant la possibilité de se lier à des anticorps monoclonaux de souris, spécifiques de VHC, utilisés dans un essai de détection d'une infection par VHC.

Les auteurs de l'invention décrite dans la demande de brevet WO2003/095968 ont rendu artificiellement différents, par modification de structure, certains épitopes des antigènes cibles utilisés pour capturer les anticorps. Les épitopes ainsi modifiés sont alors détruits. Simultanément, les anticorps utilisés pour la capture et/ou la détection des antigènes sont eux choisis de telle sorte qu'ils reconnaissent précisément des épitopes non modifiés présents sur les antigènes du patient, et qu'ils ne puissent ainsi pas se lier aux antigènes modifiés, qui ne présentent plus ces mêmes épitopes. Puisque les épitopes ne sont plus identiques, il n'existe donc plus de compétition entre les anticorps utilisés pour capturer et/ou détecter l'antigène de VHC et les anticorps du patient. La demande de brevet WO2003/095968 décrit plus précisément un peptide de capside du VHC muté en au moins deux sites épitopiques distincts, et notamment le peptide 1-75 (G34-G44-G47).

Ansaldi et al, 2006, Journal of Viral Hepatitis, 13(1):5-10 utilise également le test Monolisa HCV Ag-Ab ULTRA de la société BIO-RAD, combinant détection de la capside et des anticorps anti-capside à l'aide de ce peptide de capside 1-75 (G34-G44-G47). Alzahrani, 2008, Journal of Medical Virology, 80(1) :603-606 et Dean&Perry, « Evaluation of Murex HCV Ag/Ab combination", Health protection agency, 1er février 2007, se rapportent au test Murex HCV Ag/Ab de la société Abbott, qui ne comprend pas de peptide de capside muté. La demande de brevet WO2008/070727 et l'article Bailin et al, 2010, Clinical and Vaccine Immunology, 17(3) :1040-1047 décrivent la génération d'anticorps chimériques anti-NS3, NS4, N5 et des antigènes de capside, et mentionnent des épitopes de capside. La demande de brevet WO00/31130 rapporte que le peptide 2-45 de capside de VHC comporte des épitopes conformationnels et que ce peptide perd sa réactivité lorsqu'on fait une délétion des acides aminés 36-45.

### Résumé de l'invention :

Les inventeurs proposent maintenant un procédé de détection d'une infection par le VHC, encore plus avantageux.

Tout en évitant le problème d'interférence, le procédé présente une excellente sensibilité, permet une détection précoce, tout en permettant de suivre l'évolution sérologique du patient après la séroconversion.

Plus précisément, l'invention fournit un procédé de détection in vitro d'une infection par un virus de l'hépatite C (VHC) dans un échantillon biologique, comprenant la détection simultanée de la protéine de capside de VHC, et d'un anticorps dirigé contre ladite protéine de capside, ce procédé utilisant, pour capturer les anticorps anti-capside, un peptide comprenant un fragment antigénique dérivé de la capside de VHC tronquée.

Ainsi un objet de l'invention est un procédé de détection in vitro d'une infection par un virus de l'hépatite C (VHC) dans un échantillon biologique, comprenant la détection simultanée de la protéine de capside de VHC, et d'un anticorps dirigé contre ladite protéine de capside, présents dans l'échantillon biologique, procédé comprenant a) la mise en présence de l'échantillon biologique avec un anticorps de capture dirigé contre ladite protéine de capside, et un antigène de capture capable de capturer les anticorps anti-protéine de capside présents dans l'échantillon; b) l'incubation du mélange dans des conditions permettant la formation de complexes antigènes-anticorps ; c) la révélation des complexes antigènes-anticorps formés, qui met éventuellement en oeuvre un anticorps de détection, marqué, capable de se lier à la protéine de capside capturée et/ou éventuellement aussi un antigène de détection, marqué, capable de se lier à l'anticorps dirigé contre la capside capturé; caractérisé en ce que ledit antigène de capture est un peptide consistant en un fragment antigénique dérivé de la capside de VHC allant au maximum de l'acide aminé 1 à l'acide aminé 44 de la séquence SEQ ID NO : 1, ledit fragment antigénique comprenant, ou consistant en, i) une séquence allant des acides aminés 6 à 37 de la protéine de capside de VHC de séquence SEQ ID NO :1, et présentant une mutation ponctuelle de l'acide aminé en position 34.

Un autre objet de l'invention est un peptide comprenant un tel fragment antigénique.

Un autre objet de l'invention est une trousse utile pour la détection d'une infection par un virus VHC dans un échantillon biologique, comprenant un tel peptide, en tant qu'antigène de capture des anticorps anti-capside de VHC.

### Description détaillée de l'invention :

### Définitions

Le terme « virus de l'hépatite C » ou « VHC » couvre ici toutes les souches, tous les types, sous-types et génotypes du virus responsable de l'hépatite C. Le procédé de l'invention vise en effet à détecter toute infection par le VHC, quels que soient son origine et son génotype. Ceci comprend en particulier les types et sous-types bien connus du virus circulant en Europe, aux Etats-Unis, au Japon, etc... (c'est à dire les 6 génotypes majeurs : 1,2, 3, 4,5, 6 et leurs sous-types 1a, 1b, 3a,...). Voir Stuyver et al. (1994) ; Bukh (1995).

La protéine de capside de VHC est une protéine de 191 acides aminés, de séquence SEQ ID NO :1 :

Sauf indication contraire, les acides aminés sont localisés dans la protéine de capside en référence à la séquence SEQ ID NO :1 qui est une séquence consensus du génotype 1 (sous-types 1a, 1b, 1c).

Plusieurs épitopes de la protéine de capside ont été identifiés. On connaît notamment les épitopes localisés entre l'acide aminé 16 et l'acide aminé 40.

Par l'expression « allant au maximum de l'acide aminé 1 à l'acide aminé 44 », on entend que le peptide d'au maximum 44 acides aminés, qui ne s'étend pas au-delà de l'acide aminé 44 de la séquence SEQ ID NO :1, mais peut être éventuellement plus court, pour autant qu'il contienne la séquence 6-37 (toujours en référence à SEQ ID NO :1).

Dans le contexte de l'invention, un « échantillon biologique » est de préférence constitué par un liquide biologique, tel que du sang, plasma, sérum, de l'urine, du liquide céphalorachidien, de la salive, etc...

Le terme « anticorps » se réfère à tout anticorps entier ou fragment fonctionnel d'un anticorps comprenant ou consistant en au moins un site de reconnaissance antigénique, permettant audit anticorps de se lier à au moins un déterminant antigénique d'un composé antigénique. A titre d'exemple de fragments d'anticorps on peut citer les fragments Fab, Fab', F (ab') 2 ainsi que les chaînes scFv (Single chain variable fragment), dsFv (Double-stranded variable fragment), etc... Ces fragments fonctionnels peuvent notamment être obtenus par génie génétique.

La production d'anticorps monoclonaux ou de sérums polyclonaux monospécifiques utiles dans le cadre de l'invention relève de techniques conventionnelles, qui sont détaillées plus loin.

Par « anticorps de capture », on entend un anticorps ou une partie d'anticorps, de préférence fixé sur une phase solide, qui est capable de retenir un antigène du VHC présent dans un échantillon biologique, par liaison affine.

La présence des anticorps et antigènes dans l'échantillon biologique est révélée par des marqueurs spécifiques. S'agissant de la détection de l'antigène, l'invention prévoit notamment une détection à l'aide d'au moins un « anticorps de détection ». Un tel « anticorps de détection », marqué, est capable de se lier à l'antigène capturé, par liaison affine, en reconnaissant un site épitopique, différent de celui reconnu par l'anticorps de capture. S'agissant de la détection des anticorps, on peut utiliser notamment des anticorps anti-immunoglobuline, ou anti-isotype, marqués, par exemple des anti-immunoglobulines G dans un format ELISA indirect ou des antigènes marqués dans un format sandwich.

L'anticorps de capture et/ou l'anticorps de détection reconnaît l'épitope naturel de la portion 33-36 de la protéine de capside de VHC.

Le terme « marqué » se réfère aussi bien à un marquage direct (par le biais d'enzymes, radioisotopes, fluorochromes, composés luminescents, etc) qu'à un marquage indirect (par exemple, par le biais d'anticorps eux- mêmes marqués de manière directe ou à l'aide de réactifs d'une « paire d'affinité » marquée, telle que, mais non exclusivement, la paire avidine marquée-biotine, etc.).

Par « antigène de capture », on entend un fragment antigénique isolé, de préférence fixé sur une phase solide, qui est capable d'être reconnu par des anticorps anti- VHC et de permettre une liaison affine avec ces derniers.

Par « antigène de détection », on entend un antigène marqué. Il permet soit de détecter par compétition l'antigène capturé, soit de détecter des anticorps par méthode sandwich antigène-anticorps-antigène classique, encore appelée méthode « sandwich double antigène » (Maiolini et al. (1978)).

Le terme « spécifique » ou « spécifiquement », quand il se réfère à une reconnaissance ou une liaison spécifique d'un anticorps pour un antigène, signifie que l'anticorps interagit avec l'antigène sans interaction substantielle avec d'autres antigènes, ou si l'on parle de reconnaissance « spécifique » avec un épitope, par reconnaissance quasi-exclusive de cet épitope. Des constantes d'association supérieures à 10⁸ L. mol⁻¹ sont préférables.

Le terme « homologue » se réfère à un peptide comprenant un fragment antigénique dérivé de la capside de VHC d'au maximum 44 acides aminés. Ce fragment antigénique comprend une séquence différant de la séquence allant au maximum des acides aminés 1 à 44 de la protéine de capside de VHC, par une substitution conservative d'un ou plusieurs acides aminés, ou une délétion d'un ou plusieurs acides aminés à l'extrémité N-terminale (acides aminés 1 à 5) et /ou C-terminale (acides aminés 38 à 44).

### Anticorps et antigènes de capture et de détection

Les anticorps utilisés dans la présente invention sont des anticorps spécifiques de l'antigène, et, pour cette raison, sont des anticorps monoclonaux ou des anticorps polyclonaux monospécifiques, c'est à dire qu'ils ne reconnaissent spécifiquement qu'un épitope.

Le procédé de l'invention vise plus particulièrement à détecter des anticorps anti-capside, et utilisent des antigènes de capture qui fixent les anticorps anti-capside présents dans l'échantillon biologique.

Le peptide destiné à capturer les anticorps anti-capside comprend un fragment antigénique dérivé de la capside de VHC allant au maximum des acides aminés 1 à 44, ledit fragment antigénique comprenant une séquence allant des acides aminés 6 à 37 de la protéine de capside de VHC. Il est donc entendu que l'antigène de capture selon l'invention est dépourvu des acides aminés 45 à 191 (qui sont normalement présents dans la protéine de capside du VHC). Selon un mode de réalisation particulier, la séquence d'acides aminés du peptide utile comme antigène de capture selon l'invention comprend ou consiste en 32 à 44 acides aminés, par exemple consiste en une séquence de 44 acides aminés.

Le peptide présente au moins une mutation ponctuelle de l'acide aminé en position 34. Il peut s'agir d'une substitution par n'importe quel acide aminé non conservatif. Pour l'acide aminé 34 on utilise de préférence différent d'un résidu isoleucine, leucine, methionine, phénylalanine ou alanine. De préférence, on choisit un acide aminé non chargé. De préférence l'acide aminé valine en position 34 est substitué par une glycine.

Du fait de la mutation du peptide antigénique de capture, il n'y a pas interférence entre la réaction antigène immobilisé-anticorps anti-capside de l'échantillon, et la réaction anticorps immobilisé-antigène de capside de l'échantillon.

Selon un mode de réalisation préféré, l'anticorps de capture immobilisé sur la phase solide est un anticorps dirigé contre l'épitope naturel 33-36 de la protéine de capside, tandis que l'anticorps de détection en c1), marqué directement ou indirectement, est dirigé contre un épitope différent des épitopes naturels présents dans la portion 33 à 36, plus généralement 6 à 37, de ladite protéine de capside du VHC. Par exemple il peut s'agir d'un anticorps dirigé contre l'épitope 44-47 de la capside.

Selon un autre mode de réalisation préféré, l'anticorps de capture immobilisé sur la phase solide est un anticorps dirigé contre un épitope différent des épitopes naturels présents dans la portion 33 à 36, plus généralement 6 à 37, de ladite protéine de capside du VHC - par exemple il peut s'agir d'un anticorps dirigé contre l'épitope 44-47 de la capside-, tandis que l'anticorps de détection en c1), marqué directement ou indirectement, est un anticorps dirigé contre l'épitope naturel 33-36 de la protéine de capside.

Selon un mode de réalisation particulier, l'antigène de capture est un peptide consistant en la séquence 1 à 44 de la protéine de capside de VHC, et présentant une mutation ponctuelle de l'acide aminé en position 34, de préférence une substitution en glycine.

Un exemple préféré est donc le peptide de séquence SEQ ID NO :2.

₁MSTNPKPQRKTKRNTNRRPQDVKFPGGGQIVGG**G₃₄**YLLPRRGPRL

Un peptide homologue, consistant en une séquence telle que définie ci-dessus, mais tronquée en outre de 1 à 5 acides aminés du côté N-terminal ou C-terminale, peut aussi être utilisé. On peut ainsi utiliser le peptide 6-37, présentent une mutation ponctuelle d'au moins un acide aminé dans la séquence 33-36, et de préférence de l'acide aminé en position 34. De préférence il s'agit du peptide de séquence SEQ ID NO :3.

₆KPQRKTKRNTNRRPQDVKFPGGGQIVGG**G₃₄**YLL₃₇

Des peptides ne se distinguant des peptides ci-dessus que par substitution conservatives, sont également décrits ici.

L'expression "substitution conservative" exprime tout remplacement d'un résidu acide aminé par un autre, sans altération de la conformation générale ni de l'antigénicité du peptide. La substitution conservative inclut, sans s'y limiter, le remplacement par un acide aminé ayant des propriétés similaires (comme par exemple la forme, la polarité, le potentiel de liaison hydrogène, l'acidité, la basicité, l'hydrophobicité et autres). Les acides aminés ayant des propriétés similaires sont bien connus dans l'art. Par exemple, l'arginine, l'histidine et la lysine sont des acides aminés hydrophiles basiques et peuvent être interchangeables. De la même façon, l'isoleucine, un acide amine hydrophobe, peut être remplacé par une leucine, une méthionine ou une valine. Les acides aminés hydrophiles neutres pouvant se substituer les uns les autres incluent l'asparagine, la glutamine, la sérine et la thréonine. Par "substitué" et "modifié " l'invention comprend les acides aminés ayant été altéré ou modifié par rapport à un acide aminé trouvé dans la nature.

Ainsi, dans le contexte de la présente invention, une substitution conservative est une substitution d'un acide aminé par un autre ayant des propriétés similaires. Des exemples de substitutions conservatives sont donnés dans le tableau 1 ci-dessous :

**Tableau 1. Substitutions conservatives I**

| **Caractéristique de la chaîne latérale** | **Acide aminé** |
|---|---|
| Non polaire | G A P I L V |
| Polaire non chargée | C S T M N Q |
| Polaire chargée | D E K R |
| Aromatique | H F W Y |
| autre | N Q D E |

Selon Lehninger, 1975, les acides aminés conservatifs peuvent également être groupés comme illustré dans le tableau 2 ci-dessous :

**Table 2. Substitutions conservatives II**

| **Caractéristique de la chaîne latérale** | **Acide aminé** |
|---|---|
| Non polaire (hydrophobe) | |
| A. Aliphatique : | A L I V P |
| B. Aromatique : | F W |
| C. Contenant un sulfuryle : | M |
| D. Autre | G |
| | |
| Polaire non chargée | |
| A. Hydroxyle: | S T Y |
| B. Amides : | N Q |
| C. Sulfhydryle: | C |
| D. Autre | G |
| Chargée positivement (basique) | K R H |
| Chargée négativement (acide) | D E |

Encore une autre alternative de substitution conservative est présentée dans le tableau 3 ci-dessous:

**Table 3. Substitutions Conservatives III**

| **Résidu d'origine** | **Exemple de substitution** |
|---|---|
| Ala (A) | Val (V), Leu (L), Ile(I) |
| Arg (R) | Lys (K), Gln (Q), Asn (N) |
| Asn (N) | Gln (Q), His (H), Lys (K), Arg (R) |
| Asp (D) | Glu (E) |
| Cys(C) | Ser (S) |
| Gln (Q) | Asn (N) |
| Glu (E) | Asp (D) |
| His (H) | Asn (N), Gln (Q), Lys (K), Arg (R) |
| Ile(I) | Leu (L), Val (V), Met (M), Ala (A), Phe (F) |
| Leu (L) | Ile(I), Val (V), Met (M), Ala (A), Phe (F) |
| Lys (K) | Arg (R), Gln (Q), Asn (N) |
| Met (M) | Leu (L), Phe (F), Ile(I) |
| Phe (F) | Leu (L), Val (V), Ile(I), Ala (A) |
| Pro (P) | Gly (G) |
| Ser (S) | Thr (T) |
| Thr (T) | Ser (S) |
| Trp (W) | Tyr (Y) |
| Tyr (Y) | Trp (W), Phe (F),Thr (T), Ser (S) |
| Val (V) | Ile(I), Leu (L), Met (M), Phe (F), Ala (A) |

Les peptides conformes à l'invention peuvent être préparés par toutes les techniques classiques de synthèse peptidique, à savoir notamment par synthèse chimique ou recombinaison génétique. Dans un mode de réalisation préféré, les peptides sont obtenus par synthèse chimique. De préférence encore, les peptides sont obtenus soit par condensation successive des résidus d'acides aminés dans l'ordre requis, soit par condensation des résidus sur un fragment préalablement formé et contenant déjà plusieurs acides aminés dans l'ordre approprié, ou encore par condensation de plusieurs fragments préalablement préparés, en prenant soin de protéger, au préalable, toutes les fonctions réactives portées par les résidus d'acides aminés, excepté les fonctions amine et carboxyle engagées dans la liaison peptidique lors de la condensation, et notamment par la technique de synthèse en phase solide de Merrifield, qui est avantageuse pour des raisons de pureté, de spécificité antigénique, d'absence de produits secondaires non désirés et pour sa facilité de mise en oeuvre (Merrifield, (1963) ; R. C. Sheppard (1971) ; Atherton et al. (1989)). Comme synthétiseur automatique, on peut utiliser le synthétiseur"9050 Plus Pep Synthesizer"de Millipore, le synthétiseur "Pioneer"de Perseptive, le synthétiseur "433A"de ABI (Applied Biosystems Inc.) ou le synthétiseur «Symphony » de Rainin. Les peptides peuvent également être obtenus par synthèse en phase homogène.

Le peptide antigénique destiné à capturer les anticorps anti-capside peut être prolongé à son extrémité N-Terminale par un bras espaceur

Un objet de l'invention est aussi un tel conjugué peptidique comprenant le peptide antigénique, lié de manière covalente, à son extrémité N-Terminale, à un bras espaceur..

Par « bras espaceur», on entend une molécule qui est de préférence un peptide, de préférence de 1 à 8 acides aminés de préférence fonctionnalisé, c'est-à-dire présentant des fonctions thiol, hydrazide, aldehyde, ou une biotine, par exemple. Il s'agit.généralement d'un peptide de 1 à 8 acides aminés, qui peut comprendre des acides aminés non naturels, tels les acides ω-aminés. De préférence ce bras espaceur contient au moins un résidu cystéine. Selon un mode de réalisation préféré, le bras espaceur est C-Hx, où C est une cystéine et Hx l'acide 6-aminohexanoïque. Selon un autre mode de réalisation, le bras espaceur est le peptide de séquence CGG.

Le peptide de capture peut être couplé de manière covalente à lui-même ou il peut être couplé à une molécule porteuse via le bras espaceur .Le bras espaceur permet ainsi d'optimiser l'accrochage du peptide antigénique sur une surface solide, une protéine ou un polymère fonctionnalisé soluble et/ou permet de conjuguer plusieurs molécules antigéniques. De préférence, la molécule porteuse est une protéine comme l'albumine bovine sérique (BSA), ou l'ovalbumine. Selon un autre mode de réalisation, le peptide est lié, via le bras espaceur, à un amino-dextran.

Ainsi, selon un mode de réalisation particulier, le conjugué peptidique comprend un bras espaceur lié de manière covalente à plusieurs peptides antigéniques, identiques ou différents. De préférence un homodimère est ainsi formé.

De manière particulièrement préférée, l'antigène de capture des anticorps anti-capside est uniquement un peptide antigénique tel que défini plus haut (par exemple le peptide de séquence SEQ ID NO :2).

Cependant, dans un mode de réalisation particulier, l'antigène de capture selon l'invention, tel que défini plus haut (par exemple le peptide de séquence SEQ ID NO :2), peut être utilisé en combinaison avec un autre antigène de capture (présent de préférence sur la même phase solide que l'antigène de capture selon l'invention), dont la séquence d'acides aminés comprend ou consiste en un fragment de la protéine de capside de VHC, allant des acides aminés 45 ou 46 à un des acides aminés compris entre 60 et 80, de préférence entre 65 et 75 ou entre 68 et 75 (toujours en référence à SEQ ID NO :1). Dans un mode de réalisation plus particulier, l'antigène de capture supplémentaire consiste en la séquence 45-65, 45-66, 45-67, 45-68, 45-69, 45-70, 45-71, 45-72, 45-73, 45-74, ou 45-75, ou encore la séquence 46-65, 46-66, 46-67, 46-68, 46-69, 46-70, 46-71, 46-72, 46-73, 46-74, ou 46-75. A titre d'exemples, on peut citer le peptide 45-65, le peptide 45-68 ou, de préférence encore, le peptide 45-75;

On peut en outre combiner la détection des anticorps anti-capside à celle d'anticorps contre une autre protéine de VHC. Divers antigènes de capture, ou de détection, peuvent être combinés ensemble. Ce mode de réalisation, qui met en oeuvre plusieurs antigènes de capture, et/ou de détection, différents, permet, par exemple, la détection simultanée d'anticorps anti-capside et d'anticorps anti- protéines non structurales, par exemple NS3 et/ou NS4 ou NS5 du VHC. Une détection simultanée des anticorps anti-capside et des anticorps anti-NS3 est particulièrement préférée. L'invention comprend également une détection simultanée d'antigène capsidique, d'anticorps anti-capside, d'anticorps anti-protéines structurales d'enveloppe E1 et/ou E2, d'antigène d'enveloppe E1 et/ou E2, et/ou d'anticorps anti-protéines non structurales NS3 et/ou NS4 ou NS5 du VHC. D'autres combinaisons envisageables font aussi partie de l'invention.

### Procédés de détection

Le procédé de l'invention comprend la détection simultanée de la protéine de capside de VHC, et d'un anticorps dirigé contre ladite protéine de capside, présents dans l'échantillon biologique, procédé comprenant a) la mise en présence de l'échantillon biologique avec un anticorps de capture dirigé contre ladite protéine de capside, et un antigène de capture capable de capturer les anticorps anti-protéine de capside présents dans l'échantillon; b) l'incubation du mélange dans des conditions permettant la formation de complexes antigènes-anticorps ; c) la révélation des complexes antigènes-anticorps formés, qui met éventuellement en oeuvre un anticorps de détection, marqué, capable de se lier à la protéine de capside capturée et/ou éventuellement aussi un antigène de détection, marqué, capable de se lier à l'anticorps dirigé contre la capside capturé.

De manière générale, et sauf indication contraire, l'antigène de capture se réfère ici aussi bien au fragment antigénique dérivé de la capside de VHC allant au maximum de l'acide aminé 1 à l'acide aminé 44, qu'à un conjugué peptidique comprenant ce fragment, comme décrit plus haut.

L'échantillon biologique peut être éventuellement traité dans une étape préalable, ou mis en présence de l'antigène de capture et de l'anticorps de capture dans des conditions favorisant l'exposition des antigènes à détecter.

Avantageusement, on traite l'échantillon par un agent dénaturant, avant la détection, et de préférence, avant sa mise en contact avec les anticorps utilisés. Cet agent dénaturant peut notamment être constitué d'un ou plusieurs détergents de type non-ionique, tel (s) que, par exemple, le Tween 20, le Triton X-100, le Nonidet P-40 (NP40) (tert-Octylphenoxy poly (oxyethylene) ethanol, encore appelé IGEPAL CA630), le n-octyl beta D-glucopyranoside, ou encore d'une solution acide.

Cet immunoessai combiné peut être réalisé selon divers formats bien connus de l'homme du métier : en phase solide ou en phase homogène ; en un temps ou en deux temps ; en méthode double sandwich (sandwich pour les deux détections d'antigènes et d'anticorps) ; ou en méthode indirecte (pour la détection d'anticorps) combinée à une méthode sandwich (pour la détection d'antigène), à titre d'exemples non limitatifs.

Selon un mode de réalisation préféré, l'anticorps de capture et l'antigène de capture sont immobilisés sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque Estapor), ou encore des tubes à essai en polystyrène ou polypropylène, ou encore une membrane de nitrocellulose, etc...

Un format d'immunoessai de type sandwich entre deux anticorps (de capture et de détection) est particulièrement avantageux pour la détection des antigènes présents dans l'échantillon biologique, tandis que les anticorps peuvent être révélés en mettant en oeuvre un antigène de capture et un conjugué marqué qui se fixe sur l'anticorps (selon un format communément désigné comme « format indirect »), par exemple la proteine A marquée ou encore un anticorps anti immunoglobuline, ou anti-isotype, marqué. On peut encore détecter avantageusement les anticorps en mettant en oeuvre un antigène de capture et un antigène marqué qui se fixent sur l'anticorps (selon un format désigné comme « sandwich antigène-anticorps-antigène » ou « sandwich double antigène »).

Un format d'immunoessai de détection des antigènes par compétition est également possible. D'autres modalités d'immunoessai sont encore envisageables et bien connues de l'homme du métier.

La détection simultanée de l'antigène de VHC et des anticorps anti-VHC selon l'invention, peut être réalisée en un seul temps, à savoir en mettant simultanément en présence l'échantillon biologique, les moyens de détection, tels que notamment le ou les anticorps de détection, en même temps que le ou les anticorps de capture et le ou les antigène (s) de capture. Dans ce cas, l'immunoessai de détection de l'antigène et l'immunoessai de détection des anticorps sont tous les deux réalisés de préférence en sandwich. De manière alternative, les moyens de détection, tels que notamment le ou les anticorps de détection, peuvent être ajoutés au mélange dans un deuxième temps, c'est à dire après que les premiers complexes antigènes-anticorps se sont formés. On parle alors d'essai en deux temps.

Selon un mode de réalisation préféré de l'invention, le procédé de détection d'une infection par le virus de l'hépatite C (VHC) dans un échantillon biologique comprend :
a) la mise en présence de l'échantillon avec un anticorps de capture de la protéine de capside du VHC et un antigène de capture des anticorps anti-capside de VHC fixés sur une phase solide ; b) l'incubation du mélange dans des conditions permettant la formation de complexes antigènes-anticorps ; c) la séparation de la phase solide et de la phase liquide ; d) la mise en contact de la phase solide avec un anticorps de détection, marqué, capable de lier l'antigène de VHC capturé, et un ou des anticorps anti-immunoglobuline, ou anti-isotype, marqué, capable de lier l'anticorps anti-VHC capturé.

Dosages ELISA, radioimmunoessais, ou toute autre technique de détection peuvent être mises en oeuvre pour révéler la présence des complexes antigènes-anticorps formés.

La détection de la présence d'antigènes ou d'anticorps dans l'échantillon biologique peut être complétée par une quantification, par exemple par mesure des signaux émis par les marqueurs (couleur, luminescence, radioactivité,...), selon les techniques standards bien connues de l'homme de métier.

### Trousses

Les trousses et réactifs utiles pour la détection d'une infection par le VHC dans un échantillon biologique, conformément au procédé de l'invention, peuvent être fournis pour une mise en pratique de l'invention facile et applicable à de nombreux échantillons biologiques.

L'invention fournit donc une trousse utile pour la détection d'une infection par un virus VHC dans un échantillon biologique, comprenant un peptide antigénique capable de capturer des anticorps anti-capside de VHC. La trousse peut comprendre en outre un moyen de détection desdits anticorps anti-VHC présents dans l'échantillon biologique et complexés audit antigène de capture, ledit moyen de détection étant de préférence un anticorps anti-immunoglobuline ou anti-isotype marqué.

De manière avantageuse, cette trousse peut contenir plusieurs antigènes et plusieurs anticorps de capture.

La trousse peut contenir en outre des antigènes d'autres protéines du virus, telles que NS3 et/ou NS4, et/ou NS5 ces antigènes étant alors destinés à capturer des anticorps anti-NS3 et/ou anti-NS4, et/ou NS5. De préférence ces antigènes sont mélangés à l'antigène de capture dérivé de la capside.

Comme cela est décrit ci-dessus, l'anticorps de capture et l'antigène de capture, peuvent être présentés de manière avantageuse sous une forme immobilisée sur une phase solide, telle qu'une microplaque.

Une trousse préférée comprend
a1) un antigène de capture qui est un peptide consistant en un fragment antigénique dérivé de la capside de VHC allant au maximum de l'acide aminé 1 à l'acide aminé 44,, ledit fragment antigénique comprenant, ou consistant en, i) une séquence allant des acides aminés 6 à 37 de la protéine de capside de VHC, et présentant au moins une mutation ponctuelle de l'acide aminé en position 34, ;
a2) de préférence également des antigènes de capture des anticorps anti-protéines non structurales comprenant tout ou partie des protéines non structurales NS3, NS4, et/ou NS5,
b) un anticorps de capture dirigé la protéine de capside du VHC;
   ledit antigène de capture et ledit anticorps de capture étant immobilisés sur une phase solide ; et
c1) un anticorps de détection, marqué,
c2) un anticorps ou des anticorps anti-immunoglobulines ou éventuellement un antigène de détection, marqué, qui comprend, ou est, un fragment antigénique qui un peptide consistant en un fragment antigénique dérivé de la capside de VHC allant au maximum de l'acide aminé 1 à l'acide aminé 44,, ledit fragment antigénique consistant enune séquence allant des acides aminés 6 à 37 de la protéine de capside de VHC, et présentant une mutation ponctuelle d'au moins un acide aminé aux positions 33 à 36.

L'anticorps de capture et/ou l'anticorps de détection reconnaît l'épitope naturel de la portion 33-36 de la protéine de capside de VHC.

Selon un mode de réalisation préféré, l'anticorps de capture immobilisé sur la phase solide est un anticorps dirigé contre l'épitope naturel 33-36 de la protéine de capside, tandis que l'anticorps de détection en c1), marqué directement ou indirectement, est dirigé contre un épitope différent des épitopes naturels présents dans la portion 33 à 36, plus généralement 6 à 37, de ladite protéine de capside du VHC. Par exemple il peut s'agir d'un anticorps dirigé contre l'épitope 44-47 de la capside.

Selon un autre mode de réalisation préféré, l'anticorps de capture immobilisé sur la phase solide est un anticorps dirigé contre un épitope différent des épitopes naturels présents dans la portion 33 à 36, plus généralement 6 à 37, de ladite protéine de capside du VHC - par exemple il peut s'agir d'un anticorps dirigé contre l'épitope 44-47 de la capside-, tandis que l'anticorps de détection en c1), marqué directement ou indirectement, est un anticorps dirigé contre l'épitope naturel 33-36 de la protéine de capside.

La trousse peut encore comprendre, en outre, un détergent, plus particulièrement un détergent non-ionique connu de l'homme du métier.

Les exemples suivants illustrent l'invention sans en limiter la portée.

### EXEMPLES : Détection d'une infection par le virus de l'hépatite C

### Matériels pour le protocole d'essai:

1) Phase solide choisie : microplaque Maxisorp, Nunc (Danemark).
2) Conjugué anticorps monoclonal anti-capside-biotine(« acm-POD ») :
   un conjugué de l'anticorps monoclonal anti-capside, Acm 2 marqué à la peroxydase est préparé selon le protocole décrit dans la demande de brevet EP 0 752 102.
   Un autre anticorps monoclonal anti-capside, Acm 1 est aussi utilisé.
3) Conjugué anticorps polyclonal de souris anti-IgG (Fc_{γ}) humaines-POD : ce conjugué est obtenu chez Jackson Immunoresearch Laboratories, USA (format ELISA indirect). Conjugué peptide capside marqué à la biotine (format sandwich)
4) Diluants des 1^{ère} et 2ème étapes des protocoles selon l'invention :
   Diluant de la 1^{ère} étape : Tampon Tris, NaCl 0.05M, à pH 6,7 additionné d'IGEPAL CA 630 (Sigma) à 0,25%.
   Diluant de la 2^{ème} étape : Tampon citrate (50 mM), à pH 6,7, glycérolé à 20%.
5) Solution de révélation: la solution de révélation était composée
   5a) d'un tampon substrat : Solution d'acide citrique (0,075M), et d'acétate de sodium (0,1M), à pH 4,0, contenant de l'H₂O₂ à 0,015% et du Diméthylsulfoxyde (DMSO) (PROLABO) à 4%, et
   5b) d'un chromogène : Solution contenant de la tetraméthylbenzidine (TMB) (8 mM), (Sigma).

### Méthodes :

### Protocole de détection simultanée de l'antigène capside et des anticorps (anti-capside et anti NS3, NS4) du virus de l'hépatite C dans un échantillon (sérum ou plasma)

Le principe du test est basé sur une méthode immunoenzymatique de type sandwich pour la détection de l'antigène, et de type indirect, pour la détection des anticorps.

Il repose sur les étapes suivantes :
Une solution de sensibilisation est tout d'abord réalisée:
- avec un mélange d'antigènes VHC : un peptide muté CHx-1-44 (G34) (capside) comprenant la séquence SEQ ID n°2 et deux protéines recombinées produites par *Escherichia coli* à partir de clones sélectionnés dans les régions non structurales NS3 (AA 1192-1657), et NS4 (AA 1694-1735) et
- avec un anticorps monoclonal anti-capside (Acm 1),
en tampon Tris 0,5M, pH 7,4.

Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01M, pH 7.4) contenant 0,1% de Tween 20, puis saturées par addition d'un tampon phosphate (0,01 M, pH 7) contenant 5% de saccharose, 25% de lait écrémé (Candia™, France, ou tout autre lait écrémé équivalent du commerce) et 10mM d'EDTA.

Dans chaque cupule, on distribue successivement 100 µl de diluant de 1^{ère} étape) contenant l'anticorps monoclonal anti-capside Acm 2 marqué à la peroxydase, puis 50 µl d'échantillon (sérum ou plasma).

Le milieu réactionnel est mis à incuber à 37°C pendant 1,5 heure. Les antigènes de capside du VHC éventuellement présents se fixent sur l'anticorps monoclonal de la phase solide Acm 1 et forment des complexes avec l'anticorps monoclonal anti-capside Acm 2 marqué à la peroxydase. De même, si des anticorps anti-VHC sont présents, ils se lient aux antigènes fixés sur la phase solide.

Les plaques sont ensuite lavées (3 fois) à l'aide d'une solution de lavage (tampon Tris NaCl 0,01 M, pH 7,4 additionné de Tween 20 à 0,1%).

Dans chaque cupule, on distribue 100µl de diluant de 2^{ème} étape contenant les anticorps anti-IgG humaines marqués à la peroxydase ou le peptide capside marqué à la biotine. Le milieu réactionnel est mis à incuber à température ambiante (18-24°C) pendant 30 minutes. Les anticorps anti-IgG humaines marqués ou le peptide capside marqué biotine se fixent à leur tour aux anticorps spécifiques retenus sur la phase solide.

Les plaques sont ensuite lavées (5 fois) à l'aide d'une solution de lavage (tampon Tris NaCl 0,01 M, pH 7,4 additionné de Tween 20 à 0,1%). Le conjugué anti-IgG humaines non lié est ainsi éliminé.

Dans chaque cupule, on distribue 100 µl d'une solution de révélation (tampon substrat + chromogène). On laisse la réaction se développer à l'obscurité pendant 30 minutes à température ambiante (18-24°C).

On distribue ensuite 100 µl de solution d'arrêt (H₂SO₄ 1N) dans chaque cupule. Après arrêt de la réaction, la lecture de la densité optique est effectuée au spectrophotomètre à 450/620 nm.

### Définition de la valeur-seuil :

La valeur seuil a été déterminée après analyse statistique des données de spécificité et sensibilité en utilisant la courbe de ROC (Receiver Operating Characteristic) (Berck et Schultz, (1986)).

L'étude de spécificité a porté sur 5000 échantillons de sujets sains et l'étude de sensibilité sur des échantillons positifs VHC (notamment des débuts de séroconversion) de panels commerciaux : BBI (Boston Biomedica Company, USA), Impath (USA), Serologicals (USA), Nabi (USA), ProMedDx (USA)).

La valeur seuil est calculée pour chaque plaque à partir du signal obtenu sur un témoin positif, divisé par un coefficient X, constant, spécifique du test. Elle est d'environ 0.45 unité de DO (Densité Optique) dans l'exemple présenté en format indirect.

### Exemple 1 : Détection d'anticorps anti-capside dans des échantillons (sérums ou plasmas) négatifs en antigène VHC

Trois panels d'échantillons de séroconversion PHV 905, 907 et 914 (c'est à dire deux échantillons consécutifs de sérums ou plasmas, prélevés chez deux patients après infection par le VHC ou en cours de séroconversion) commercialisés (Seracare Life Sciences / BBI Diagnostics, USA) ont été testés suivant le protocole sandwich. Le peptide capside 1-44 G34 (SEQ ID NO : 2) a alors été marqué à la biotine et le complexe révélé par de la streptavidine-peroxydase. Il s'agit donc d'une détection anti-capside unique.

Ces échantillons ont été trouvés positifs avec une valeur seuil calculée par la moyenne des trois échantillons négatifs plus 6 déviations standard (DO : 0.077)

**Tableau 1 :**

| **Echantillon** | **Densité optique** | |
|---|---|---|
| 905-08 | 0.058 | négatif |
| 905-09 | 0.209 | **positif** |
| 907-05 | 0.480 | **positif** |
| 907-06 | 0.694 | **positif** |
| 914-08 | 0.409 | **positif** |
| 914-09 | 0.824 | **positif** |
| Neg 1 | 0.043 | négatif |
| Neg 2 | 0.048 | négatif |
| Neg 3 | 0.044 | négatif |

L'invention permet donc de détecter chez des sujets contaminés par le VHC des échantillons positifs en anticorps anti-capside

### Exemple 2 : Comparaison de performances et classement de diverses techniques sur des sérums de séroconversion positifs en anticorps et/ou antigène

9 panels de séroconversion commercialisés (Seracare Life Sciences / BBI, USA ; Impath, USA), présentant des spécificités différentes ont été testés avec le test selon l'invention en suivant le protocole indiqué, et les résultats ont été comparés avec une série de tests commercialisés. Cette comparaison tient compte du nombre de jours de retard de détection par rapport à la détection d'ARN (test PCR). Ainsi la trousse présentant la plus petite somme est la plus performante en précocité de détection.

Ces panels présentent une positivité en antigène et/ ou anticorps dirigés contre les protéines NS3 et capside du virus VHC (caractérisation réalisée par le test RIBA commercialisé par la société Ortho Clinical Diagnostics).

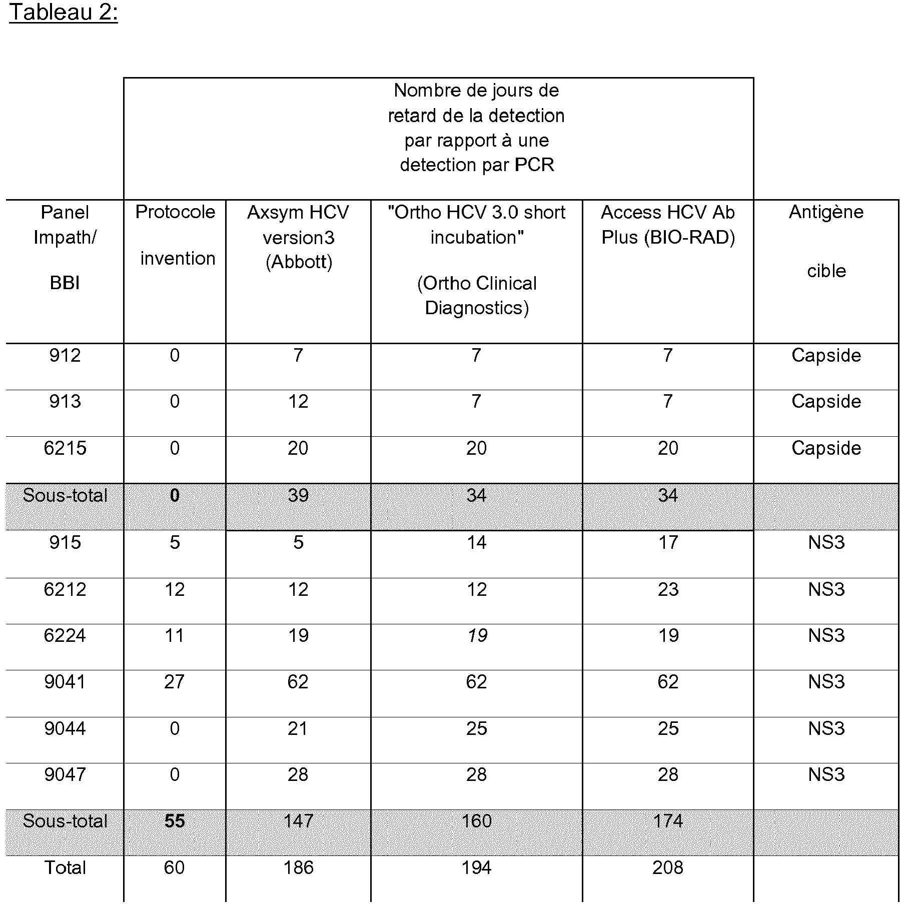

L'invention permet de détecter des échantillons positifs en anticorps et en antigène, de façon plus précoce qu'un test anticorps.

### REFERENCES BIBLIOGRAPHIQUES

- Atherton et al. (1989)"solid phase peptide synthesis, a practical approach", IRL Press, , Oxford University Press, pp. 25-34
- Bukh, Semin. Liver Dis. (1995) 15 : 41-63 ;
- Maiolini et al., (1978) Journal of Immunological Methods, 20, pp. 25-34
- Merrifield (1963) J. Amer. Chem. Soc,, 85, pp. 2149-2154
- Sheppard, dans « Peptides 1971 », Nesvadba H (ed.) North Holland, Amsterdam, p. 111
- Stuyver et al. (1994), P. N. A. S. USA, 91, pp. 10134-10138

## Revendications

1. Procédé de détection in vitro d'une infection par un virus de l'hépatite C (VHC) dans un échantillon biologique, comprenant la détection simultanée de la protéine de capside de VHC, et d'un anticorps dirigé contre ladite protéine de capside, présents dans l'échantillon biologique, procédé comprenant a) la mise en présence de l'échantillon biologique avec un anticorps de capture dirigé contre ladite protéine de capside, et un antigène de capture capable de capturer les anticorps anti-protéine de capside présents dans l'échantillon; b) l'incubation du mélange dans des conditions permettant la formation de complexes antigènes-anticorps ; c) la révélation des complexes antigènes-anticorps formés, qui met éventuellement en oeuvre un anticorps de détection, marqué, capable de se lier à la protéine de capside capturée et/ou éventuellement aussi un antigène de détection, marqué, capable de se lier à l'anticorps anti-capside capturé; **caractérisé en ce que** ledit antigène de capture est un peptide consistant en un fragment antigénique dérivé de la capside de VHC allant au maximum de l'acide aminé 1 à l'acide aminé 44 de la séquence SED ID NO :1, ledit fragment antigénique comprenant, ou consistant en une séquence allant des acides aminés 6 à 37 de la protéine de capside de VHC de séquence SED ID NO :1, et présentant une mutation ponctuelle de l'acide aminé 34.

2. Procédé selon la revendication 1, dans lequel l'antigène de capture est un peptide consistant en la séquence 1 à 44 de la protéine de capside de VHC, et présentant une mutation ponctuelle de l'acide aminé en position 34.

3. Procédé selon l'une des revendications 1 ou 2, dans lequel l'acide aminé en position 34 est un résidu glycine.

4. Procédé selon l'une quelconque des revendications 1 à 3, dans lequel ledit anticorps de capture et ledit antigène de capture sont immobilisés sur une phase solide, la phase solide pouvant contenir en outre au moins un antigène dérivé d'une protéine de VHC qui n'est pas la capside, de préférence NS3 ou NS4.

5. Procédé selon l'une quelconque des revendications 1 à 4, dans lequel l'anticorps de détection est ajouté au mélange après que les complexes antigènes-anticorps se sont formés.

6. Procédé selon l'une quelconque des revendications 1 à 5, dans lequel l'anticorps de détection, et/ou l'antigène de détection, est mis en présence de l'échantillon biologique en même temps que l'anticorps de capture et l'antigène de capture.

7. Peptide consistant en un fragment antigénique dérivé de la capside de VHC allant au maximum de l'acide aminé 1 à l'acide aminé 44 de la séquence SEQ ID NO :1, ledit fragment antigénique comprenant, ou consistant en, une séquence allant des acides aminés 6 à 37 de la protéine de capside de VHC de séquence SEQ ID NO :1, et présentant une mutation ponctuelle de l'acide aminé 34.

8. Peptide selon la revendication 7, consistant en la séquence 1 à 44 de la protéine de capside de VHC, et présentant une mutation ponctuelle de l'acide aminé en position 34.

9. Peptide selon l'une des revendications 7 ou 8 dans lequel l'acide aminé en position 34 est un résidu glycine.

10. Conjugué peptidique comprenant le peptide de l'une des revendications 7 à 9, lié de manière covalente, à son extrémité N-Terminale, à un bras espaceur, qui est de préférence C-Hx, où C est une cystéine et Hx l'acide 6-aminohexanoïque.

11. Conjugué peptidique selon la revendication 10, dans lequel le peptide tel que défini à l'une des revendications 7 à 9 est couplé de manière covalente à lui-même ou à une molécule porteuse via le bras espaceur.

12. Trousse utile pour la détection d'une infection par un virus VHC dans un échantillon biologique, comprenant un peptide tel que défini à l'une des revendications 7 à 9, en tant qu'antigène de capture des anticorps anti-capside de VHC, ladite trousse pouvant comprendre, outre l'antigène de capture, un moyen de détection desdits anticorps anti-VHC présents dans l'échantillon biologique et complexés audit antigène de capture, ledit moyen de détection étant de préférence un anticorps anti-immunoglobuline ou anti-isotype marqué.

13. Trousse selon la revendication 12, comprenant
a1) un antigène de capture des anticorps anti-capside de VHC qui est un peptide tel que défini à l'une des revendications 7 à 9;
a2) de préférence également des antigènes de capture des anticorps anti-protéines non structurales comprenant tout ou partie des protéines non structurales NS3, NS4, et/ou NS5,
b) un anticorps de capture dirigé la protéine de capside du VHC;
ledit antigène de capture et ledit anticorps de capture étant immobilisés sur une phase solide ; et
c1) un anticorps de détection, marqué,
c2) un anticorps ou des anticorps anti-immunoglobulines ou éventuellement un antigène de détection, marqué, qui est un peptide tel que défini à l'une des revendications 7 à 9.

14. Trousse selon l'une des revendications 12 ou 13, comprenant un anticorps de capture des anticorps anti-capside de VHC, immobilisé sur la phase solide, dirigé contre l'épitope naturel 33-36 de la protéine de capside, et un anticorps de détection marqué directement ou indirectement, est dirigé contre un épitope différent des épitopes naturels présents dans la portion 33 à 36, plus généralement 6 à 37, de ladite protéine de capside du VHC.

15. Trousse selon l'une des revendications 12 ou 13, comprenant un anticorps de capture des anticorps anti-capside de VHC, immobilisé sur la phase solide, dirigé contre un épitope différent des épitopes naturels présents dans la portion 33 à 36, plus généralement 6 à 37, de ladite protéine de capside du VHC, et un anticorps de détection marqué directement ou indirectement, dirigé contre l'épitope naturel 33-36 de ladite protéine de capside du VHC.

## Patentansprüche

1. In vitro Verfahren zum Nachweis einer Infektion durch einen Hepatitis-C-Virus (VHC) in einer biologischen Probe, umfassend den gleichzeitigen Nachweis des VHC-Capsidproteins und eines gegen besagtes Capsidprotein gerichteten Antikörpers, die in der biologischen Probe vorhanden sind, wobei das Verfahren umfasst: a) Inkontaktbringen der biologischen Probe mit einem Einfangantikörper, der gegen besagtes Capsidprotein gerichtet ist, und einem Einfangantigen, das in der Lage ist, die in der biologischen Probe vorhandenen Anti-Capsidprotein-Antikörper einzufangen; b) Inkubation der Mischung unter Bedingungen, die die Bildung von Antikörper-Antigen-Komplexen erlauben; c) Nachweis der gebildeten Antigen-Antikörper-Komplexe, gegebenenfalls unter Verwendung eines markierten Nachweisantikörpers, der in der Lage ist, an das eingefangene Capsidprotein zu binden und/oder gegebenenfalls auch eines markierten Nachweisantigens, das in der Lage ist, an den eingefangenen Anti-Capsid-Antikörper zu binden, **dadurch gekennzeichnet, dass** besagtes Einfangantigen ein Peptid ist, das aus einem Antigenfragment besteht, das sich vom VHC-Capsid ableitet und höchstens von Aminosäure 1 bis Aminosäure 44 der Sequenz SEQ ID NO: 1 reicht, wobei besagtes Antigenfragment eine Sequenz von Aminosäure 6 bis 37 des VHC-Capsid-Proteins von SEQ ID NO: 1 umfasst oder aus dieser besteht, und eine Punktmutation der Aminosäure 34 aufweist.

2. Verfahren gemäß Anspruch 1, wobei das Einfangantigen ein Peptid ist, das aus der Sequenz 1 bis 44 des VHC-Capsidproteins besteht und eine Punktmutation der Aminosäure in Position 34 aufweist.

3. Verfahren gemäß einem der Ansprüche 1 oder 2, wobei die Aminosäure in Position 34 ein Glycinrest ist.

4. Verfahren gemäß einem der Ansprüche 1 bis 3, wobei besagter Einfangantikörper und besagtes Einfangantigen auf einer Festphase immobilisiert sind, wobei die Festphase außerdem wenigstens ein Antigen enthalten kann, das von einem VHC-Protein, das nicht vom Capsid ist, vorzugsweise von NS3 oder NS4 abgeleitet ist.

5. Verfahren gemäß einem der Ansprüche 1 bis 4, wobei der Nachweisantikörper der Mischung nach Bildung der Antikörper-Antigen-Komplexe zugefügt wird.

6. Verfahren gemäß einem der Ansprüche 1 bis 5, wobei der Nachweisantikörper und/oder das Nachweisantigen mit der biologischen Probe zur gleichen Zeit wie der Einfangantikörper und das Einfangantigen in Kontakt gebracht wird.

7. Peptid, das aus einem vom VHC-Capsid abgeleiteten Antigenfragment besteht und höchstens von Aminosäure 1 bis Aminosäure 44 der Sequenz SEQ ID NO: 1 reicht, wobei besagtes Antigenfragment eine Sequenz, die von Aminosäuren 6 bis 37 des VHC-Caspsidproteins von Sequenz SEQ ID NO: 1 reicht, umfasst oder aus dieser besteht, und eine Punktmutation der Aminosäure 34 aufweist.

8. Peptid gemäß Anspruch 7, das aus der Sequenz 1 bis 44 des VHC-Capsidproteins besteht und eine Punktmutation der Aminosäure in Position 34 aufweist.

9. Peptid gemäß einem der Ansprüche 7 oder 8, wobei die Aminosäure in Position 34 ein Glycinrest ist.

10. Peptidkonjugat, umfassend das Peptid nach einem der Ansprüche 7 bis 9, das kovalent an seinem N-Terminus an einen Spacer gebunden ist, der vorzugsweise C-Hₓ ist, wo C ein Cystein ist und Hₓ Aminohexansäure ist.

11. Peptidkonjugat gemäß Anspruch 10, wobei das Peptid, wie in einem der Ansprüche 7 bis 9 definiert, mit sich selbst oder an ein Trägermolekül über den Spacer kovalent gekoppelt ist.

12. Kit zur Verwendung für den Nachweis einer Infektion durch einen VHC-Virus in einer biologischen Probe, umfassend ein Peptid, wie in einem der Ansprüche 7 bis 9 definiert, als Einfangantigen der Anti-VHC-Capsid-Antikörper, wobei besagtes Kit, außer dem Einfangantigen, ein Nachweismittel besagter Anti-VHC-Antikörper umfassen kann, die in der biologischen Probe vorhanden sind und mit besagtem Einfangantigen komplexiert werden, wobei besagtes Nachweismittel vorzugsweise ein markierter Anti-Immunglobulin- oder Anti-Isotyp-Antikörper ist.

13. Kit gemäß Anspruch 12, umfassend
a1) ein Einfangantigen der Anti-VHC-Capsid-Antikörper, das ein Peptid ist, wie in einem der Ansprüche 7 bis 9 definiert;
a2) vorzugsweise ebenfalls Einfangantigene der Anti-Nichtstrukturprotein-Antikörper, umfassend alle oder einige der Nichtstrukturproteine NS3, NS4 und/oder NS5;
b) einen Einfangantikörper, der gegen das VHC-Capsidprotein gerichtet ist;
wobei besagtes Einfangantigen und besagter Einfangantikörper auf einer Festphase immobilisiert sind; und
c1) einen markierten Nachweisantikörper;
c2) einen oder mehrere Anti-Immunglobulin-Antikörper oder gegebenenfalls ein markiertes Nachweisantigen, das ein wie in einem der Ansprüche 7 bis 9 definiertes Peptid ist.

14. Kit gemäß einem der Ansprüche 12 oder 13, umfassend einen Einfangantikörper der Anti-VHC-Capsid-Antikörper, der auf einer Festphase immobilisiert ist und gegen das natürliche Epitop 33-36 des Capsidproteins gerichtet ist, und einen direkt oder indirekt markierten Nachweisantikörper, der gegen ein anderes Epitop als die natürlichen Epitope gerichtet ist, die im Abschnitt 33-36, allgemeiner 6 bis 37 besagten VHC-Capsidproteins vorhanden sind.

15. Kit gemäß einem der Ansprüche 12 oder 13, umfassend einen Einfangantikörper der Anti-VHC-Capsid-Antikörper, der auf einer Festphase immobilisiert ist und gegen ein anderes Epitop als die natürlichen Epitope gerichtet ist, die im Abschnitt 33-36, allgemeiner 6 bis 37 besagten VHC-Capsidproteins vorhanden sind, und einen direkt oder indirekt markierten Nachweisantikörper, der gegen das natürliche Epitop 33-36 besagten VHC-Capsidprotein-Antikörpers gerichtet ist.

## Claims

1. An *in vitro* process for detecting hepatitis C virus (HCV) infection in a biological sample, comprising simultaneously detecting HCV capsid protein, and an antibody against said capsid protein, present in the biological sample, the process comprising a) contacting the biological sample with a capture antibody against said capsid protein, and a capture antigen capable of capturing the anti-capsid protein antibodies present in the sample; b) incubating the mixture under conditions permitting the formation of antigen-antibody complexes; c) revealing the antigen-antibody complexes formed, optionally using a labeled detection antibody capable of binding to the captured capsid protein and/or optionally also a labeled detection antigen capable of binding to the captured anti-capsid antibody; **characterized in that** said capture antigen is a peptide consisting of an antigenic fragment derived from the HCV capsid ranging at most from amino acid 1 to amino acid 44 of the sequence SED ID NO: 1, said antigenic fragment comprising, or consisting of, a sequence from amino acids 6 to 37 of the HCV capsid protein of sequence SED ID NO: 1, and having a point mutation of amino acid 34.

2. The process as claimed in claim 1, wherein the capture antigen is a peptide consisting of the sequence 1 to 44 of the HCV capsid protein, and having a point mutation of amino acid 34.

3. The process as claimed in one of claims 1 or 2, wherein the amino acid at position 34 is a glycine residue.

4. The process as claimed in any one of claims 1 to 3, wherein said capture antibody and said capture antigen are immobilized on a solid phase, the solid phase which may further contain at least one antigen derived from an HCV protein which is not the capsid, preferably NS3 or NS4.

5. The process as claimed in any of claims 1 to 4, wherein the detection antibody is added to the mixture after the antigen-antibody complexes have formed.

6. The process as claimed in any one of claims 1 to 5, wherein the detection antibody, and/or the detection antigen, is brought into contact with the biological sample together with the capture antibody and the capture antigen.

7. A peptide consisting of an HCV capsid-derived antigenic fragment ranging at most from amino acid 1 to amino acid 44 of the sequence SEQ ID NO: 1, said antigenic fragment comprising, or consisting of, a sequence ranging from amino acids 6 to 37 of the HCV capsid protein of the sequence SEQ ID NO: 1, and having a point mutation of amino acid 34.

8. The peptide as claimed in claim 7, consisting of the sequence 1 to 44 of the HCV capsid protein, and having a point mutation of amino acid 34.

9. The peptide as claimed in one of claims 7 or 8, wherein the amino acid at position 34 is a glycine residue.

10. A peptide conjugate comprising the peptide of one of claims 7 to 9, covalently bonded, at its N-terminus, to a spacer arm, which is preferably C-Hx, wherein C is cysteine and Hx is 6-aminohexanoic acid.

11. The peptide conjugate as claimed in claim 10, wherein the peptide as defined in any of claims 7 to 9 is covalently coupled to itself or to a carrier molecule via the spacer arm.

12. A kit useful for detecting HCV infection in a biological sample, comprising a peptide as defined in one of claims 7 to 9, as HCV antibody capture antigen, said kit which may comprise, in addition to the capture antigen, a means for detecting said anti-HCV antibodies present in the biological sample and complexed to said capture antigen, said detection means preferably being a labeled anti-immunoglobulin or anti-isotype antibody.

13. The kit as claimed in claim 12, comprising
a1) an anti-HCV capsid antibodies capture antigen which is a peptide as defined in one of claims 7 to 9;
a2) preferably also antibody-capture antigens against non-structural proteins comprising all or part of the non-structural proteins NS3, NS4, and/or NS5,
b) a capture antibody against the HCV capsid protein; sai
d capture antigen and said capture antibody being immobilized on a solid phase; and
c1) a labeled detection antibody,
c2) an antibody or anti-immunoglobulin antibodies or optionally a labeled detection antigen which is a peptide as defined in one of claims 7 to 9.

14. The kit as claimed in one of claims 12 or 13, comprising an anti-HCV capsid antibodies capture antibody, immobilized on the solid phase, against the natural epitope 33-36 of the capsid protein, and a detection antibody, directly or indirectly labeled, is against an epitope different from the natural epitopes present in the portion 33 to 36, more generally 6 to 37, of said HCV capsid protein.

15. The kit as claimed in one of claims 12 or 13, comprising an anti-HCV capsid antibodies capture antibody, immobilized on the solid phase, against an epitope different from the natural epitopes present in the portion 33 to 36, more generally 6 to 37, of said HCV capsid protein, and a directly or indirectly labeled detection antibody against the natural epitope 33-36 of said HCV capsid protein.


### SEQUENCE LISTING

<110> BIO-RAD Innovations
<120> Procédé de détection d'une infection par le virus de l'hépatite C
<130> B1280
<160> 3
<170> PatentIn version 3.3
<210> 1
   <211> 191
   <212> PRT
   <213> Hepatitis C virus
<400> 1
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Ser Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala
               180                 185                 190
<210> 2
   <211> 44
   <212> PRT
   <213> Hepatitis C virus
<400> 2
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Gly Tyr Leu Leu Pro Arg Arg Gly Pro Arg Leu
           35                  40
<210> 3
   <211> 32
   <212> PRT
   <213> Hepatitis C virus
<400> 3
   Lys Pro Gln Arg Lys Thr Lys Arg Asn Thr Asn Arg Arg Pro Gln Asp
   1               5                   10                  15
   Val Lys Phe Pro Gly Gly Gly Gln Ile Val Gly Gly Gly Tyr Leu Leu
               20                  25                  30